(19) Europäisches Patentamt
European Patent Office
Office européen des brevets

(11) **EP 4 083 587 A1**

(12) **EUROPEAN PATENT APPLICATION**

(43) Date of publication:
**02.11.2022 Bulletin 2022/44**

(21) Application number: **22170849.8**

(22) Date of filing: **29.04.2022**

(51) International Patent Classification (IPC):
**G01K 13/02** (2021.01)    **G01J 5/00** (2022.01)
**G01K 1/02** (2021.01)    **G01N 7/00** (2006.01)
**G01N 33/00** (2006.01)    **G01F 23/00** (2022.01)
**G01K 17/10** (2006.01)    **A61L 2/07** (2006.01)
**A61L 2/28** (2006.01)

(52) Cooperative Patent Classification (CPC):
**G01K 13/02; G01J 5/00; G01K 1/026; G01K 17/10; G01N 7/00; G01N 25/00; G01N 33/00;** A61L 2/07; A61L 2/28; G01F 1/05; G01F 1/3209; G01F 1/66

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2021  US 202163182008 P**

(71) Applicant: **3M Innovative Properties Company Saint Paul, MN 55133-3427 (US)**

(72) Inventors:
• **ABELS, Axel**
  **Neuss (DE)**
• **SCHUMACHER, Knut**
  **Neuss (DE)**
• **WISCHNEPOLSKI, Waleri**
  **Neuss (DE)**

(74) Representative: **Vossius & Partner Patentanwälte Rechtsanwälte mbB Siebertstraße 3 81675 München (DE)**

(54) **STEAM QUALITY SENSING DEVICE AND METHOD FOR MONITORING A STEAM QUALITY**

(57)    A sensing device for monitoring a steam quality of a steam sterilant in a chamber and a sterilizing system including the sensing device are provided. The sensing device sensing includes a sensor tube having an open end and a closed end. The open end is configured to receive the steam sterilant from the chamber. The sensing device further includes a housing at least partially receiving the sensor tube therein. The housing includes an inlet and an outlet. The housing defines a flow passage between the inlet and the outlet. The inlet of the housing is configured to receive a coolant within the flow passage and the outlet of the housing is configured to discharge the coolant from the flow passage. The sensing device further includes a first temperature sensor and a second temperature sensor. Each of the first and second temperature sensors is disposed in fluid communication with the flow passage.

*FIG. 1*

## Description

### Technical Field

The present disclosure relates generally to sterilization, and more particularly, relates to a sensing device, a sterilizing system, and a method for use with a sensing device.

### Background

Sterilization of medical and hospital equipment may not be effective until a steam sterilant has been in contact with all surfaces of materials being sterilized for a proper combination of time, temperature, and steam quality. In steam sterilizers, such as pre-vacuum steam sterilizers and gravity displacement steam sterilizers, the process of sterilization is conducted in three main phases. In the first phase, air is removed, including air trapped within any porous materials being processed. The second phase is a sterilizing stage, in which a load (i.e., the articles being sterilized) is subjected to steam under pressure for a recognized, predetermined combination of time and temperature to effect sterilization. The third phase is a drying phase in which condensation formed during the first two phases is removed by evacuating the chamber.

Any air that is not removed from the sterilizer during the air removal phase of the cycle or which leaks into the sterilizer during a sub atmospheric pressure stage due to, for example, faulty gaskets, valves or seals, may form air pockets within any porous materials present. Such air pockets may create a barrier to steam penetration, thereby preventing adequate sterilizing conditions being achieved for all surfaces of the load during the sterilizing phase. For example, these air pockets may prevent the steam from reaching interior layers of materials, such as hospital linens or fabrics. In some other examples, these air pockets may prevent the steam from penetrating hollow spaces of tubes, catheters, syringe needles, and the like. Further, non-condensable gas (generally air) present within the sterilizer is a poor sterilant and may decrease sterilization efficacy. A percentage of non-condensable gas in the steam should be less than or equal to 3.5% by volume. Therefore, the presence of air pockets and/or non-condensable gas may affect a steam quality of the steam sterilant. As a result, proper sterilization may not occur due to reduced steam quality. A few more factors that may affect steam quality include insufficient steam supply, water quality, degassing, design of the sterilizer chamber, etc.

### Summary

In a first aspect, the present disclosure provides a sensing device for monitoring a steam quality of a steam sterilant in a chamber. The sensing device includes a sensor tube having an open end and a closed end opposite to the open end. The open end is disposed in fluid communication with the chamber and configured to receive the steam sterilant from the chamber. The sensing device further includes a housing at least partially receiving the sensor tube therein and surrounding at least a portion of the sensor tube between the open end and the closed end. The housing includes an inlet and an outlet spaced apart from the inlet. The housing defines a flow passage at least partially along a length of the sensor tube between the inlet and the outlet. The inlet of the housing is configured to receive a coolant within the flow passage and the outlet of the housing is configured to discharge the coolant from the flow passage. The sensing device further includes a first temperature sensor disposed in fluid communication with the flow passage. The first temperature sensor is configured to measure a temperature of the coolant proximal to the inlet of the housing. The sensing device further includes a second temperature sensor disposed in fluid communication with the flow passage. The second temperature sensor is configured to measure a temperature of the coolant proximal to the outlet of the housing.

In a second aspect, the present disclosure provides a sterilizing system including a steam sterilizer and the sensing device of the first aspect. The steam sterilizer defines a chamber therein. The chamber is configured to receive a steam sterilant therein. The sensor tube of the sensing device is coupled to the steam sterilizer, such that the open end of the sensor tube is disposed in fluid communication with the chamber of the steam sterilizer.

In a third aspect, the present disclosure provides a method for monitoring a steam quality of a steam sterilant in a chamber. The method includes providing a sensor tube having an open end and a closed end opposite to the open end. The method further includes at least partially receiving the sensor tube within a housing. The housing includes an inlet and an outlet spaced apart from the inlet. The method further includes receiving the steam sterilant within the sensor tube via the open end. The method further includes providing a coolant, via the inlet, to a flow passage defined by the housing. The flow passage extends at least partially along a length of the sensor tube between the inlet and the outlet. The method further includes measuring, by a first temperature sensor disposed in fluid communication with the flow passage, a temperature of the coolant proximal to the inlet of the housing. The method further includes measuring, by a second temperature sensor disposed in fluid communication with the flow passage, a temperature of the coolant proximal to the outlet of the housing.

## Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.

FIG. 1 is a schematic front view of a sterilizing system, according to an embodiment of the present disclosure;
FIG. 2 is a detailed schematic sectional view of the sterilizing system of FIG. 1, according to an embodiment of the present disclosure;
FIG. 3 is a detailed schematic sectional view of the sterilizing system of FIG. 1, according to another embodiment of the present disclosure;
FIG. 4 is a perspective view of a sensing device of the sterilizing system, according to an embodiment of the present disclosure;
FIG. 5 is a front perspective view of a sensor tube of the sensing device of FIG. 4, according to an embodiment of the present disclosure;
FIG. 6 is a schematic front view of another sterilizing system, according to an embodiment of the present disclosure;
FIG. 7 is an exploded view of a sensing device of the sterilizing system of FIG. 6, according to an embodiment of the present disclosure;
FIG. 8 illustrates a schematic front perspective view of the sensing device of FIG. 7, according to an embodiment of the present disclosure;
FIG. 9 illustrates a schematic side perspective view of the sensing device of FIG. 7, according to an embodiment of the present disclosure;
FIG. 10 illustrates a schematic front perspective view of a sensor tube of the sensing device of FIG. 7, according to an embodiment of the present disclosure;
FIG. 11 illustrates a schematic front perspective view of the sensor tube of the sensing device of FIG. 7, according to another embodiment of the present disclosure;
FIG. 12 is a schematic block diagram illustrating a processor communicably coupled to various sensors of the sensing devices of FIGS. 4 and 7, according to an embodiment of the present disclosure;
FIG. 13 is a schematic partial front view of a portion of a sensor tube of the sensing devices of FIGS. 4 and 7, according to an embodiment of the present disclosure;
FIG. 14 is a flowchart for a method for monitoring a steam quality of a steam sterilant, according to an embodiment of the present disclosure; and
FIG. 15 is a schematic block diagram illustrating a cloud system associated with the sterilizing systems of FIGS. 1 and 6, and the processor of FIG. 12, according to an embodiment of the present disclosure.

## Detailed Description

In the following description, reference is made to the accompanying figures that form a part thereof and in which various embodiments are shown by way of illustration. It is to be understood that other embodiments are contemplated and may be made without departing from the scope or spirit of the present disclosure. The following detailed description, therefore, is not to be taken in a limiting sense.

Steam sterilizers are widely used in medical centers and hospitals to sterilize medical equipment. Frequent testing or monitoring of steam quality may be essential to ensure a safe use of the medical equipment in a medical treatment. In other words, regular testing may have to be conducted to check effectiveness of air removal during air removal phase of the sterilization process, prior to subjecting the steam to a given load (i.e., medical equipment). One of the conventional ways to monitor steam quality of the steam sterilant is Bowie-Dick test. In general, the Bowie-Dick test uses an indicator sheet and a test pack having stack of freshly laundered towels. In some cases, the indicator sheet is a chemical indicator sheet. In some cases, the indicator sheet is a bio indicator sheet. In some cases, the test pack used in the Bowie-Dick test includes a disposable test pack.

Although the Bowie-Dick type test is generally recognized as an adequate procedure for determining the steam quality of the steam sterilant or efficacy of the air removal stage of steam sterilization process, it may face some challenges. Since the test pack is not preassembled, it must be constructed every time the procedure is used to monitor the steam quality. Further, the testing procedure may be somewhat inconsistent because varying factors, such as laundering, prehumidification, towel thickness and wear, and the number of towels used, may alter the test results. Further, the preparation, assembly and use of the towel pack may be time consuming and cumbersome.

Moreover, results of the Bowie-Dick type test (with chemical indicators as well as bio indicators) include only two possible outcomes, i.e., pass or fail. Therefore, in case of a failed Bowie-Dick test, a technician or user may not be able to find any information about the root cause of the test failure. In other words, a user may not receive any information about the reason of poor steam quality of the steam sterilant. This may cause a delay to identify any growing problem in the steam sterilizer and warn an operator in advance prior to a severe problem. This may result in a breakdown of the steam sterilizer at any time in future.

Some of the conventional ways to monitor steam quality include usage of physical indicators. The physical indicators may record temperature and pressure of steam to determine an information about the steam quality. Such physical indicators may also provide an information indicating a root cause of low steam quality. However, after testing steam quality of a steam sterilant in a steam sterilizer, a physical indicator may take a relatively long time to cool down (recovery time) before the physical indicator can be used to run steam monitoring tests for subsequent cycles. Therefore, the physical indicator may not be suitable for conducting a high number of successive tests to monitor steam quality in one or more steam sterilizers. Hence, using the physical indicator may limit a total count of tests that can be conducted in a given time period.

The present disclosure relates to a sensing device, a sterilizing system including the sensing device, and a method for use with the sensing device.

The sensing device of the present disclosure is used for monitoring a steam quality of a steam sterilant in a chamber. The sensing device includes a sensor tube having an open end and a closed end opposite to the open end. The open end is disposed in fluid communication with the chamber and configured to receive the steam sterilant from the chamber. The sensing device further includes a housing at least partially receiving the sensor tube therein and surrounding at least a portion of the sensor tube between the open end and the closed end. The housing includes an inlet and an outlet spaced apart from the inlet. The housing defines a flow passage at least partially along a length of the sensor tube between the inlet and the outlet. The inlet of the housing is configured to receive a coolant within the flow passage and the outlet of the housing is configured to discharge the coolant from the flow passage. The sensing device further includes a first temperature sensor disposed in fluid communication with the flow passage. The first temperature sensor is configured to measure a temperature of the coolant proximal to the inlet of the housing. The sensing device further includes a second temperature sensor disposed in fluid communication with the flow passage. The second temperature sensor is configured to measure a temperature of the coolant proximal to the outlet of the housing.

The sterilizing system includes a steam sterilizer defining a chamber therein. The chamber is configured to receive a steam sterilant therein. The sterilizing system further includes the sensing device. The sensor tube is coupled to the steam sterilizer, such that the open end of the sensor tube is disposed in fluid communication with the chamber of the steam sterilizer.

As the sensing device is an integral part of the steam sterilizer, a user may determine the steam quality of the steam sterilant or efficacy of the air removal stage of steam sterilization process in an easy and convenient manner. By using an appropriate algorithm, the sensing device may be used as an apparatus to conduct Bowie-Dick test to monitor the steam quality of the steam sterilant received within the steam sterilizer. Further, the sterilizing system including the sensing device is a built-in and a stand-alone system. Therefore, the sterilizing system of the present disclosure may not require insertion of any additional components, such as indicator sheets, into the steam sterilizer and then removal of the additional components to determine the steam quality. This may enable rapid testing of the steam quality of the steam sterilant. Consequently, the disclosed sterilizing system including the sensing device may increase an efficiency and decrease an overall cost and a complexity of a testing process to determine the steam quality of the steam sterilant. The sterilizing system may reduce or eliminate recurring costs associated with conventional testing methods, since no disposable components are required for testing. As the sensing device is coupled to the steam sterilizer, there may be no need for separate storage of the testing unit, i.e., sensing device. Further, there may be no possibility of misplacing the sensing device.

Moreover, the sensing device may be used for a longer time period and for a greater number of test cycles as compared to conventional test units. In some cases, there may not be any limit to the number of test cycles in which the sensing device can be used. Therefore, the sterilizing system including the sensing device may be an overall sustainable setup with low operational costs. The sensing device may be used for a next test cycle immediately after a current test cycle is completed.

Therefore, the disclosed sterilizing system including the sensing device may require minimal or no service/recovery time between two consecutive test cycles. Further, mounting of the sensing device outside the steam sterilizer may ensure that the sensing device does not consume any space within the steam sterilizer. Consequently, a space within the steam sterilizer may be optimally utilized for receiving a load or medical equipment.

Further, the disclosed sensing device including the sensor tube may be provided with a greater cooling capacity by the coolant flowing within the flow passage. In some cases, the coolant flowing within the flow passage may provide an unlimited heat capacity to the sensor tube. Consequently, dimensions of the sensor tube may be increased as per application requirements to achieve an improved testing sensitivity without any limitations being imposed by possible overheating. This may improve an overall sensitivity of the sensing device to the steam quality of the steam sterilant received within the sensor tube. Therefore, the sensing device may provide more accurate and precise test results while monitoring the steam quality of the steam sterilant.

Additionally, one or more output signals of the sensing device may be quantitatively related to the steam quality of the steam sterilant. Therefore, the sensing device may provide quantitative measurements related to the steam quality as opposed to a mere pass or fail indication. Such quantitative measurements can help in detecting possible causes of low steam quality.

Furthermore, most of the commercially available steam sterilizers already include one or two lead-ins extending from a sidewall or a top wall of the chamber. The disclosed sensing device may be coupled to the lead-ins of such existing steam sterilizers. Thus, the sensing device can be easily retrofitted to steam sterilizers that are already manufactured and are being currently used in the medical industry.

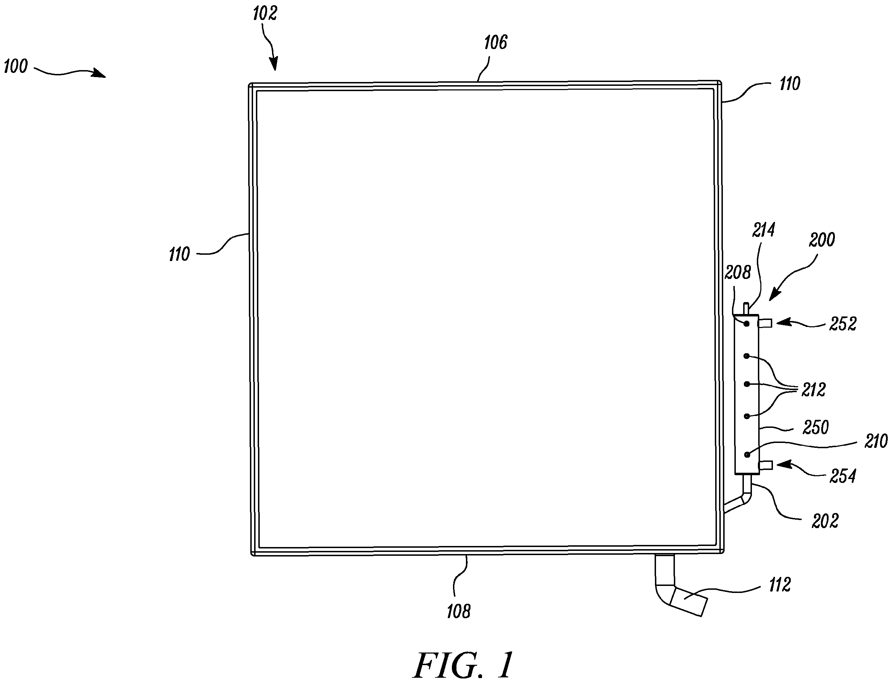

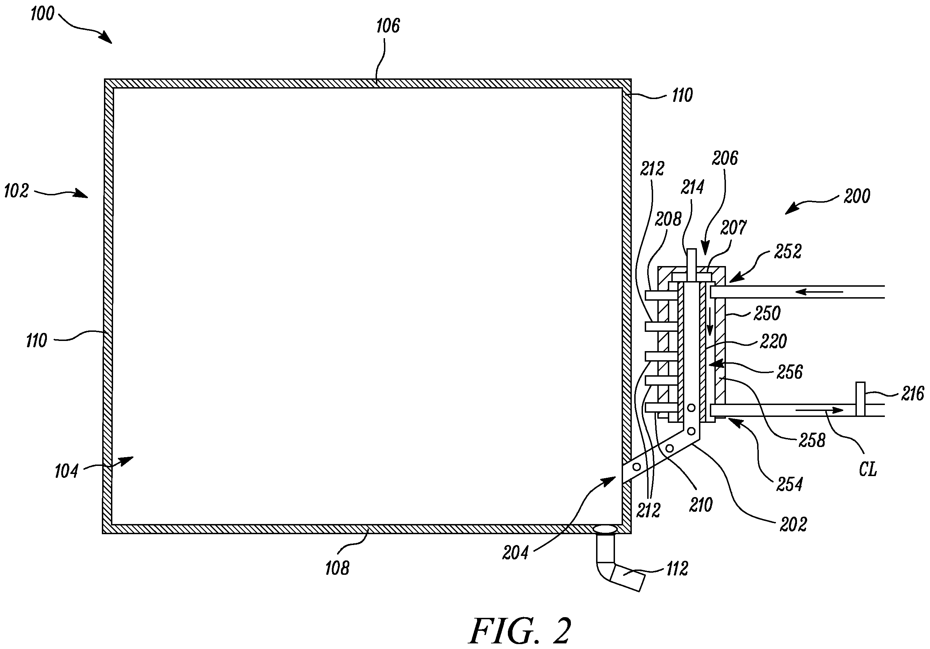

Referring now to Figures, FIG. 1 illustrates a sterilizing system 100. The sterilizing system 100 includes a steam sterilizer 102 defining a chamber 104 (shown in FIG. 2) therein. Some components of the sterilizing system 100 are not shown in FIG. 1 for the purpose of clarity. The chamber 104 is configured to receive a steam sterilant therein. When steam is used as steam sterilant, an object of sterilization process is to bring steam at an appropriate temperature into contact with all surfaces of the articles being sterilized for an appropriate period of time.

In the illustrated embodiment of FIG. 1, the steam sterilizer 102 includes a top wall 106, a bottom wall 108 opposite to the top wall 106, and a pair of side walls 110 extending between the top wall 106 and the bottom wall 108. The top wall 106, the bottom wall 108, and the sidewalls 110 define the chamber 104 therebetween. The steam sterilizer 102 further includes a drain tube 112 for removal of condensate from the chamber 104 of the steam sterilizer 102. The drain tube is coupled to the bottom wall 108 of the steam sterilizer 102.

The sterilizing system 100 further includes a sensing device 200 for monitoring a steam quality of the steam sterilant in the chamber 104. The sensing device 200 includes a sensor tube 202 having an open end 204 (shown in FIG. 2) and a closed end 206 (shown in FIG. 2) opposite to the open end 204. The sensor tube 202 of the sensing device 200 is coupled to the steam sterilizer 102. In some embodiments, the sensor tube 202 is coupled to the steam sterilizer 102 by welding. In some embodiments, the sensor tube 202 is coupled to the steam sterilizer 102 by using fasteners. In some embodiments, the sensor tube 202 is coupled to the top wall 106 or one of the sidewalls 110.

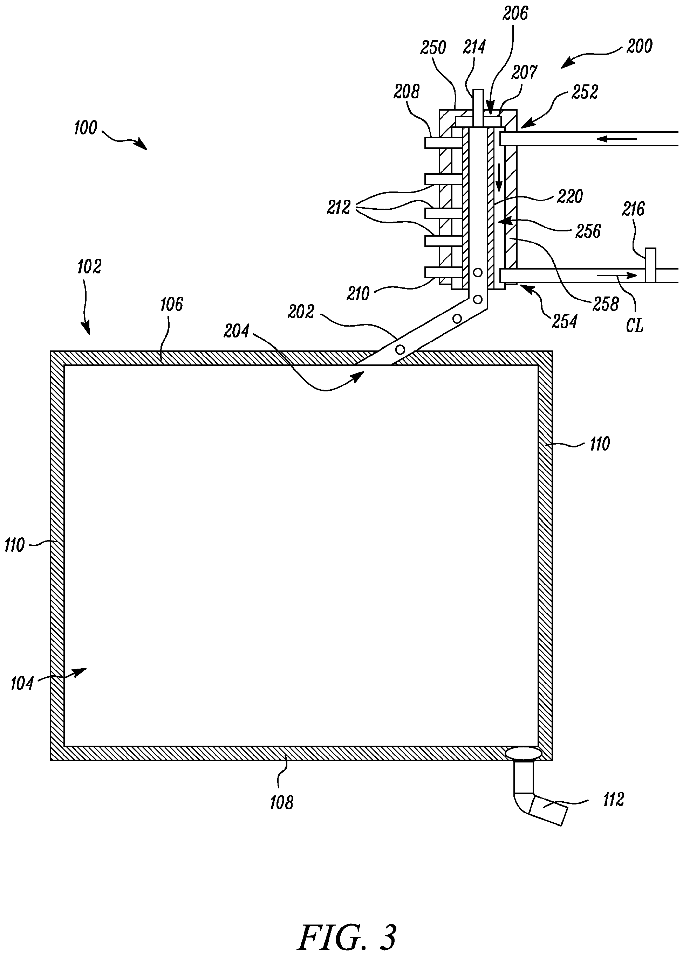

FIG. 2 is a detailed schematic sectional view of the sterilizing system 100 according to an embodiment of the present disclosure. In the illustrated embodiment of FIG. 2, the sensor tube 202 is coupled to one of the sidewalls 110 of the of the steam sterilizer 102. FIG. 3 is another detailed schematic sectional view of the sterilizing system 100 according to an embodiment of the present disclosure. In the illustrated embodiment of FIG. 3, the sensor tube 202 is coupled to the top wall 106 of the of the steam sterilizer 102. In some embodiments, the sensor tube 202 is made of stainless steel. However, the sensor tube 202 may be made of any suitable material, for example, but not limited to, a metal or a metal alloy, a plastic, a composite, and so forth.

Referring to FIGS. 1 and 2, the open end 204 of the sensor tube 202 is disposed in fluid communication with the chamber 104 of the steam sterilizer 102. The open end 204 is configured to receive the steam sterilant from the chamber 104.

The sensing device 200 further includes a housing 250. The housing 250 at least partially receives the sensor tube 202 therein and surrounds at least a portion of the sensor tube 202 between the open end 204 and the closed end 206. The housing 250 includes an inlet 252 and an outlet 254 spaced apart from the inlet 252. The housing 250 defines a flow passage 256 at least partially along a length of the sensor tube 202 between the inlet 252 and the outlet 254. The inlet 252 of the housing 250 is configured to receive a coolant within the flow passage 256 and the outlet 254 of the housing 250 is configured to discharge the coolant from the flow passage 256. In some cases, the inlet 252 of the housing 250 may receive the coolant from a heat sink (not shown). In some embodiments, the coolant includes a liquid or a gas. In some embodiments, the coolant includes a liquid, e.g., water. In the illustrated embodiments of FIGS. 2 and 3, the housing 250 includes a coolant jacket 258. The coolant jacket 258 surrounds at least a portion of the sensor tube 202. In some embodiments, the flow passage 256 may be an annular flow passage disposed at least partially around the sensor tube 202. Further, the coolant jacket 258 may have a substantially annular configuration.

In the illustrated embodiment, the flow passage 256 receives a flow of a coolant CL via the inlet 252. Further, the flow of the coolant CL is discharged from the flow passage 256 via the outlet 254.

In some embodiments, the inlet 252 of the housing 250 is disposed proximal to the closed end 206 of the sensor tube 202 and the outlet 254 of the housing 250 is disposed proximal to the open end 204 of the sensor tube 202. In some other embodiments, the inlet 252 of the housing 250 is disposed proximal to the open end 204 of the sensor tube 202 and the outlet 254 of the housing 250 is disposed proximal to the closed end 206 of the sensor tube 202.

In some embodiments, the sensing device 200 includes an insulation layer 220 disposed at least partially around the sensor tube 202 between the open end 204 and the closed end 206. Therefore, the insulation layer 220 is disposed between the flow passage 256 and a portion of the sensor tube 202. The insulation layer 220 around the sensor tube 202 may reduce heat transfer between the sensor tube 202 and the coolant. Therefore, a sensitivity of the sensor tube 202 can be adjusted by selecting appropriate material and/or dimensions of the insulation layer 220 around the sensor tube 202. The flow passage 256 may be therefore at least partially defined between the coolant jacket 258 and the insulation layer 220.

The insulation layer 220 includes a thermally insulating material. In some cases, the insulation layer 220 includes a foil or a sheet of a thermally insulating material. In some embodiments, the foil or the sheet may include a material including polyester, polypropylene, polyacrylonitrile, polyurethane, polyamide, polyimide (such as those commercially available under the trademark "Kapton" from DuPont), polyether imide, polytetrafluoroethylene (PTFE), polyvinylchloride, polycarbonate, epoxy resin, polymethyl-methacrylate, polyethylene, polystyrene, or any combinations thereof. The use of such foils or sheets may further aid in providing a well-defined heat transfer between the sensor tube 202 and the coolant and/or in controlling or optimizing the amount of heat transfer between the sensor tube 202 and the coolant. Such insulating foils or sheets preferably includes a material having a thermal conductivity which is lower than that of the sensor tube 202. Such materials may have a thermal conductivity of 5 $Wm^{-1}K^{-1}$ or less, more preferably of 1 $Wm^{-1}K^{-1}$ or less.

The sensing device 200 further includes a first temperature sensor 208 disposed in fluid communication with the flow passage 256. The first temperature sensor 208 is configured to measure a temperature of the coolant proximal to the inlet 252 of the housing 250. The sensing device 200 further includes a second temperature sensor 210 disposed in fluid communication with the flow passage 256. The second temperature sensor 210 is configured to measure a temperature of the coolant proximal to the outlet 254 of the housing 250. In some embodiments, the first temperature sensor 208 and the second temperature sensor 210 are spaced apart from each other along the length of the sensor tube 202.

With reference to FIGS. 1-3, in some embodiments, the sensing device 200 further includes at least one intermediate temperature sensor 212 disposed in fluid communication with the flow passage 256 between the first temperature sensor 208 and the second temperature sensor 210. The at least one intermediate sensor 212 is configured to measure the temperature of the coolant. In some embodiments, the at least one intermediate temperature sensor 212 includes a plurality of intermediate temperature sensors 212. There may be any number of intermediate temperature sensors 212 between the first temperature sensor 208 and the second temperature sensor 210.

In some embodiments, each of the first temperature sensor 208, the second temperature sensor 210, and the at least one intermediate temperature sensor 212 may include a thermocouple, a thermistor, an infrared sensor, and so forth.

In some embodiments, the sensing device 200 further includes a pressure sensor 214 disposed in fluid communication with the sensor tube 202. The pressure sensor 214 is configured to measure a pressure of the steam sterilant within the sensor tube 202. In some embodiments, the pressure sensor 214 may include a piezoelectric sensor, a piezoresistive sensor, a capacitive sensor, an electromagnetic sensor, and so forth. In some cases, the pressure of the steam sterilant may be determined by measuring a pressure of the steam sterilant within the steam sterilizer 102. In some cases, the pressure of the steam sterilant may be selected as a pressure of a steam sterilant in a holding phase (e.g., 3 bar) of the sterilization cycle.

In some embodiments, the sensing device 200 further includes a flow sensor 216 disposed in fluid communication with the flow passage 256 and configured to measure a flow rate of the coolant flowing through the flow passage 256. In some embodiments, the flow sensor 216 may include a vortex flow sensor, a mechanical flow sensor an ultrasonic flow sensor, and so forth.

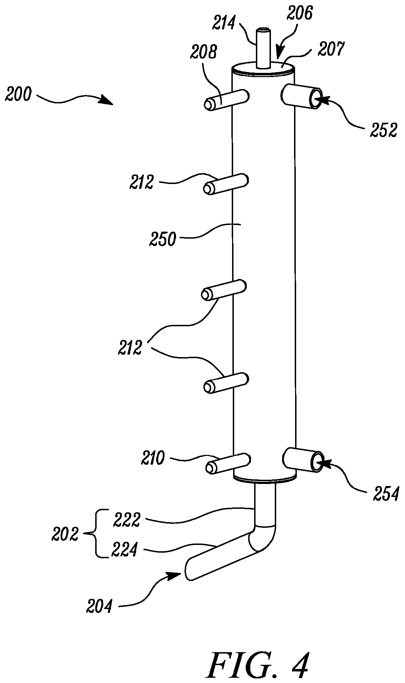

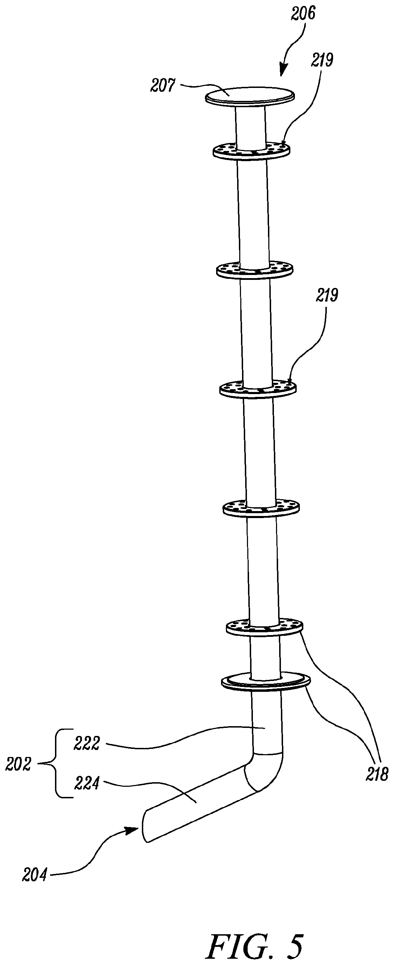

FIG. 4 illustrates a perspective view of the sensing device 200. FIG. 5 illustrates a perspective view of the sensor tube 202 of the sensing device 200 according to an embodiment of the present disclosure. With reference to FIGS. 4 and 5, in some embodiments, the sensing device 200 further includes a plurality of laminar plates 218 spaced apart from each other along the length of the sensor tube 202. Each laminar plate 218 is engaged with and disposed around the sensor tube 202. Each laminar plate 218 defines a plurality of apertures 219 therethrough. Specifically, each laminar plate 218 with the plurality of apertures 219 provides a laminar coolant flow and prevent the coolant from swirling. This may increase an accuracy of temperature measurement of the coolant by the first temperature sensor 208, the second temperature sensor 210, and the at least one intermediate sensor 212. Further, each intermediate temperature sensor 212 is fluidly disposed between two corresponding adjacent laminar plates 218.

With reference to FIGS. 4 and 5, in some embodiments, the sensor tube 202 includes a main portion 222 and an inlet portion 224 extending from the main portion 222. The main portion 222 includes the closed end 206. The inlet portion 224 includes the open end 204. Specifically, the inlet portion 224 of the sensor tube 202 is coupled to the top wall 106 or one of the sidewalls 110 of the steam sterilizer 102. Further, the inlet portion 224 is inclined relative to the main portion 222. In some embodiments, an inclination angle between the inlet portion 224 and the main portion 222 may be an oblique angle. In some embodiments, the inclination angle may be at most about 80 degrees, at most about 70 degrees, or at most about 60 degrees.

In some embodiments, the main portion 222 is at least partially surrounded by the housing 250 and the inlet portion 224 extends at least partially through the housing 250. The sensor tube further includes a cap 207 coupled to the main portion 222 for providing the closed end 206 of the sensor tube 202. In some cases, the cap 207 may also close the fluid passage 256 (shown in FIG. 2) from one end.

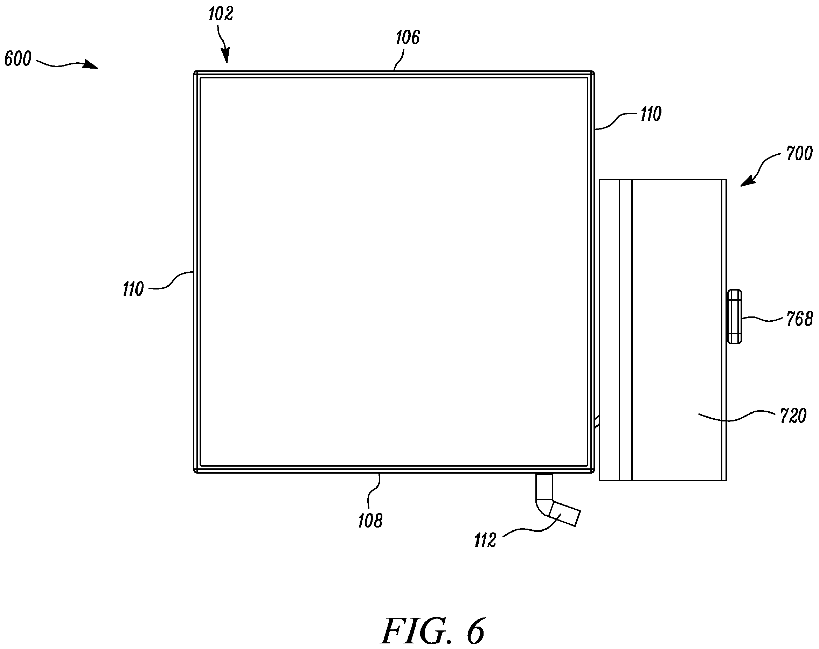

FIG. 6 illustrates a sterilizing system 600. The sterilizing system 600 includes the steam sterilizer 102 (also shown in FIG. 1). The sterilizing system 600 further includes a sensing device 700 for monitoring the steam quality of the steam sterilant in the chamber 104 (shown in FIG. 2).

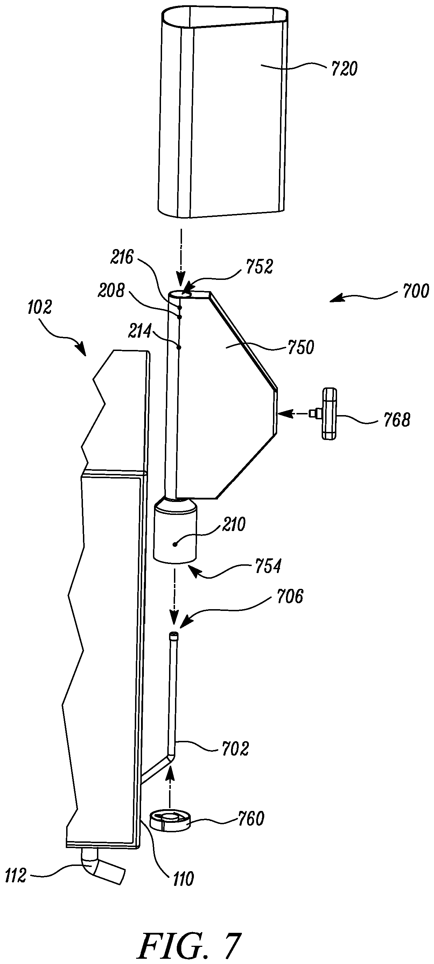

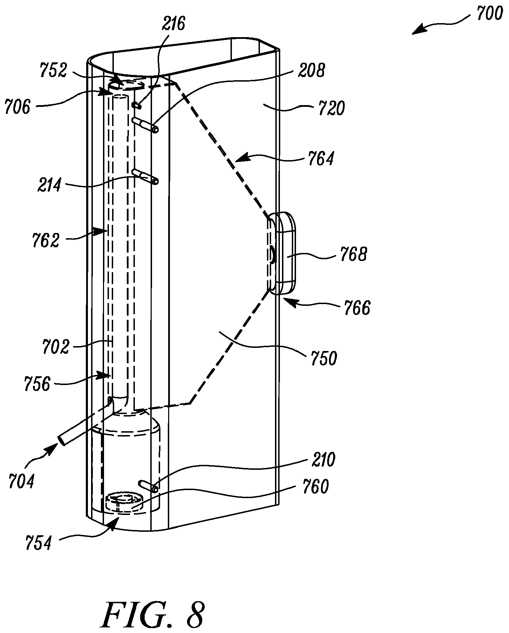

FIG. 7 illustrates an exploded view of the sensing device 700. The sensing device 700 includes a sensor tube 702 having an open end 704 (shown in FIG. 8) and a closed end 706 opposite to the open end 704. The sensor tube 702 of the sensing device 700 is coupled to the steam sterilizer 102. In some embodiments, the sensor tube 702 is coupled to the steam sterilizer 102 by welding. In some embodiments, the sensor tube 702 is coupled to the steam sterilizer 102 by using fasteners. In the illustrated embodiment of FIG. 7, the sensor tube 702 is coupled to one of the sidewalls 110 of the steam sterilizer 102. In some embodiments, the sensor tube 702 is made of stainless steel.

The open end 704 of the sensor tube 702 is disposed in fluid communication with the chamber 104 (shown in FIG. 2) of the steam sterilizer 102. The open end 704 is configured to receive the steam sterilant from the chamber 104.

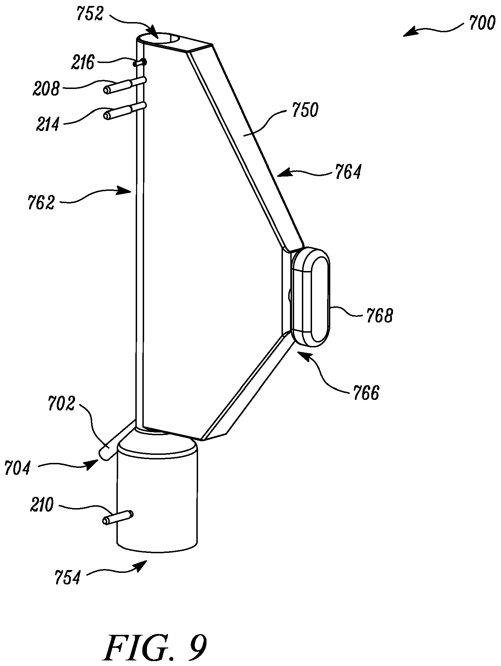

FIGS. 8 and 9 illustrate perspective views of the sensing device 700 according to an embodiment of the present disclosure. Hidden components are shown by dashed lines in FIG. 8 . Referring to FIGS. 6-9, the sensing device 700 further includes a housing 750. The housing 750 at least partially receives the sensor tube 702 therein and surrounds at least a portion of the sensor tube 702 between the open end 704 and the closed end 706. The housing 750 includes an inlet 752 and an outlet 754 spaced apart from the inlet 752. The housing 750 defines a flow passage 756 at least partially along a length of the sensor tube 702 between the inlet 752 and the outlet 754. The inlet 752 of the housing 750 is configured to receive a coolant within the flow passage 756 and the outlet 754 of the housing 750 is configured to discharge the coolant from the flow passage 756. In some embodiments, the coolant includes a liquid or a gas. In some embodiments, the inlet 752 of the housing 750 may receive air as a coolant from ambient.

In some embodiments, the inlet 752 of the housing 750 is disposed proximal to the closed end 706 of the sensor tube 702 and the outlet 754 of the housing 750 is disposed proximal to the open end 704 of the sensor tube 702. In some other embodiments, the inlet 752 of the housing 750 is disposed proximal to the open end 704 of the sensor tube 702 and the outlet 754 of the housing 750 is disposed proximal to the closed end 706 of the sensor tube 702.

In some embodiments, the sensing device 700 further includes a fan 760 disposed within the housing 750 proximal to the outlet 754. The fan 760 is configured to generate a flow of the coolant through the housing 750. In some other embodiments, the fan 760 may be disposed at other positions, for example, proximal to the inlet 752 of the housing 750.

In some embodiments, the sensing device 700 further includes an insulating case 720 at least partially receiving the housing 750 therein. The insulating case 720 includes a thermally insulating material. The insulating case 720 is not shown in FIG. 9 for illustrative purposes. The inclusion of the insulating case 720 in the sensing device 700 may reduce an amount of heat energy that can dissipate through the housing 750. This may result in a precise measurement of temperature of the coolant, and indirectly accurate monitoring of steam quality of the steam sterilant.

The insulating case 720 may include a thermally insulating material. In some cases, the insulation case 720 includes a foil or a sheet of a thermally insulating material. In some embodiments, the foil or the sheet may include a material including polyester, polypropylene, polyacrylonitrile, polyurethane, polyamide, polyimide (such as those commercially available under the trademark "Kapton" from DuPont), polyether imide, polytetrafluoroethylene (PTFE), polyvinylchloride, polycarbonate, epoxy resin, polymethyl-methacrylate, polyethylene, polystyrene, or any combinations thereof.

The sensing device 700 further includes the first temperature sensor 208 (also shown in FIG. 4) disposed in fluid communication with the flow passage 756. The first temperature sensor 208 is configured to measure a temperature of the coolant proximal to the inlet 752 of the housing 750. The sensing device 700 further includes the second temperature sensor 210 (also shown in FIG. 4) disposed in fluid communication with the flow passage 756. The second temperature sensor 210 is configured to measure a temperature of the coolant proximal to the outlet 754 of the housing 750. In some embodiments, the first temperature sensor 208 and the second temperature sensor 210 are spaced apart from each other along the length of the sensor tube 702.

In some embodiments, the sensing device 700 further includes the pressure sensor 214 (also shown in FIG. 4) disposed in fluid communication with the sensor tube 702. The pressure sensor 214 is configured to measure a pressure of the steam sterilant within the sensor tube 702. In some cases, the pressure of the steam sterilant may be determined by measuring a pressure of the steam sterilant within the steam sterilizer 102. In some cases, the pressure of the steam sterilant may be selected as a pressure of a steam sterilant in a holding phase (e.g., 3 bar) of the sterilization cycle.

In some embodiments, the sensing device 700 further includes the flow sensor 216 (also shown in FIGS. 2 and 3) disposed in fluid communication with the flow passage 756 and configured to measure a flow rate of the coolant flowing through the flow passage 756.

In some embodiments, the sensing device 700 further includes a first portion 762 and a second portion 764 extending from the first portion 762. The first portion 762 includes the inlet 752 and the outlet 754. The first portion 762 at least partially receives the sensor tube 702 therein. Further, upon coupling of the sensing device 700 to the steam sterilizer 102, the first portion 762 is proximate the steam sterilizer 102, and the second portion 764 is distal from the steam sterilizer 102. The second portion 764 includes a sensor end 766 distal to the first portion 762. In the illustrated embodiment of FIGS. 8 and 9, the second portion 764 of the housing 750 tapers from the first portion 762 to the sensor end 766.

In some embodiments, the sensing device 700 further includes a thermal imaging sensor 768 coupled to the housing 750 and configured to detect a thermal profile of the sensor tube 702. The thermal imaging sensor 768 is disposed at the sensor end of the second portion 764 of the housing 750. In some embodiments, the thermal imaging sensor 768 is an infrared camera. The thermal imaging sensor 768 may continually take pictures of the sensor tube 702 and therefore generate a thermal profile of the sensor tube 702. Thus, the thermal imaging sensor 768 may determine a height of non-condensable gas within the sensor tube 702. The height of non-condensable gas within the sensor tube 702 may be used to monitor the steam quality of the steam sterilant, which is explained later is the description.

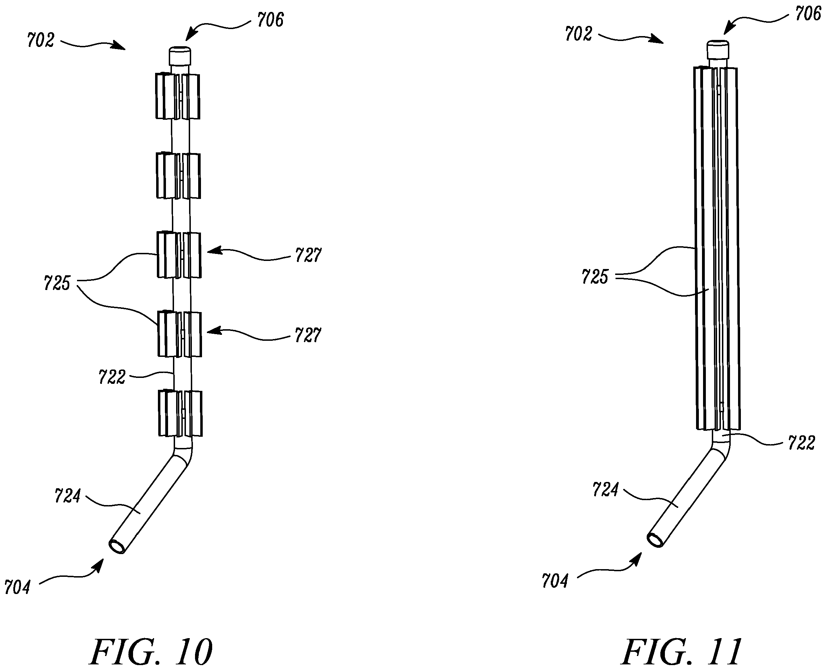

FIG. 10 illustrates a perspective view of the sensor tube 702 according to an embodiment of the present disclosure. FIG. 11 illustrates another perspective view of the sensor tube 702 according to an embodiment of the present disclosure.

In some embodiments, the sensing device 700 further includes a plurality of cooling fins 725 disposed on the sensor tube 702 at least partly along the length of the sensor tube 702. The cooling fins 725 may provide an increased surface area for heat transfer between the coolant and the sensor tube 702. In the illustrated embodiment of FIG. 10, the sensing device 700 includes a plurality of sets 727 of the cooling fins 725 disposed on the sensor tube 702. Each set 727 of the cooling fins 725 is spaced apart from an adjacent set of the cooling fins 725. Further, each set 727 includes multiple cooling fins 725 arranged around the sensor tube 702. In the illustrated embodiment of FIG. 11, the cooling fins 725 extend continuously along the length of the sensor tube 702 and are disposed around the sensor tube 702.

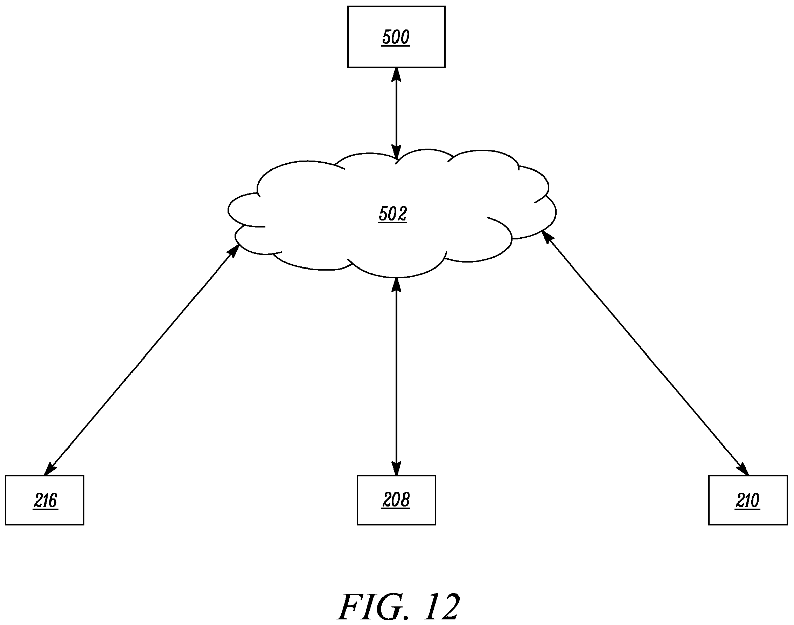

In some embodiments, each of the sensing devices 200, 700 (shown in FIGS. 1 and 6, respectively) includes a processor 500 (shown in FIG. 12). The processor 500 may be a programmable analog and/or digital device that can store, retrieve, and process data. In an application, the processor 500 may be a controller, a control circuit, a computer, a workstation, a microprocessor, a microcomputer, a central processing unit, a server, or any suitable device or apparatus.

FIG. 12 illustrates a schematic block diagram of the processor 500 communicably coupled to the first temperature sensor 208, the second temperature sensor 210, and the flow sensor 216, according to an embodiment of the present disclosure. Specifically, the processor 500 is communicably coupled to the first temperature sensor 208, the second temperature sensor 210, and the flow sensor 216 via a network 502. In some cases, the network 502 is a long-range network. In some cases, the network 502 is a short-range network. In some cases, each of the sensing devices 200, 700 may include a remote server including the processor 500. The remote server may be a computing system. In some cases, the network 502 may include a combination of wireless connections and wired connections.

In some embodiments, the processor 500 is configured to determine the steam quality of the steam sterilant based at least partially on the pressure of the steam sterilant and signals received from the first temperature sensor 208, the second temperature sensor 210, and the flow sensor 216. In some embodiments, the processor 500 is configured to determine the pressure of the steam sterilant based on signals received from the pressure sensor 214. In some embodiments, the processor 500 may also be communicably coupled to the pressure sensor 214. Specifically, in the sensor tubes 202, 702, a quantity of non-condensable gas (NCG) versus an amount of steam is determined for a selected time interval based at least partially on the pressure of the steam sterilant and the signals received from the first temperature sensor 208, the second temperature sensor 210, and the flow sensor 216. Any appropriate ratio of determined quantities of the non-condensable gas and the steam within the sensor tubes 202, 702 may serve as a good measure for steam quality of the steam sterilant.

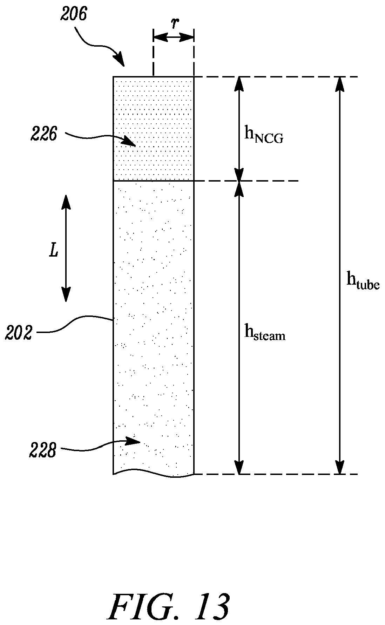

FIG. 13 illustrates a schematic front view of a portion of the sensor tube 202 of the sensing device 200 (shown in FIG. 1). Exemplary calculations will be described for monitoring the steam quality of the steam sterilant within the sensor tube 202 shown in FIGS. 1-5. However, similar equations and calculations can also be used to monitor the steam quality of the steam sterilant within the sensor tube 702 shown in FIGS. 7-9.

The sensor tube 202 is shown transparent in FIG. 13 for the purpose of illustration. Referring to FIGS. 1-5 and 13, the sensor tube 202 has a height "$h_{tube}$" between a point adjacent to the closed end 206 and a point on the sensor tube 202 adjacent to the outlet 254 (shown in FIGS. 1-5) of the housing 250. A portion 226 of the sensor tube 202 having a height "$h_{NCG}$" is filled with the non-condensable gas and a portion 228 of the sensor tube 202 having a height "$h_{steam}$" is filled with steam, not yet condensed. The portion 226 of the sensor tube 202 is proximate the closed end 206 of the sensor tube 202. The non-condensable gas is collected in the portion 226 due to continuous movement of the steam towards the closed end 206 of the sensor tube 202. Each of the heights "$h_{tube}$", "$h_{NCG}$", "$h_{steam}$" may be measured along a length "L" of the sensor tube 202.

Further, when the non-condensable gas is collected in the sensor tube 202, the non-condensable gas may insulate a part (i. e., the portion 226) of the sensor tube 202. Thus, the portion 228 of the sensor tube 202 that is filled with the steam sterilant and in thermal contact with the coolant may contribute to a total condensation power of the steam sterilant "$P_{steam}$".

In some embodiments, the processor 500 (shown in FIG. 12) determines the total condensation power of the steam sterilant "$P_{steam}$" based at least partially on the signals received from the first temperature sensor 208, the second temperature sensor 210, and the flow sensor 216. The total condensation power of the steam sterilant "$P_{steam}$" condensed in the sensor tube 202 at a given time "t" can be determined according to Equation 1 provided below:

$$P_{steam}(t) = (T_{outlet} - T_{inlet}) \cdot V_c \cdot C_c \qquad \text{(Equation 1)}$$

where, $P_{steam}$ is the total condensation power of the steam sterilant condensed in the sensor tube 202;

$T_{outlet}$ is a temperature of the coolant proximal to the outlet 254 of the housing 250;
$T_{inlet}$ is a temperature of the coolant proximal to the inlet 252 of the housing 250;
$V_c$ is a flow rate of the coolant flowing through the flow passage 256; and
$C_c$ is specific heat capacity of the coolant.

Assuming that a temperature of an inner surface of the sensor tube 202 is equal to temperature of the steam sterilant inside the sensor tube 202, the total condensation power of the steam sterilant "$P_{steam}$" at a given time "t" can be determined according to Equation 2 provided below:

$$P_{Steam}(t) = (T_{steam} - T_c) \cdot \frac{A}{R} \qquad \text{(Equation 2)}$$

where, $T_{steam}$ is a temperature of the steam sterilant;

$T_c$ is an average temperature of the coolant;
A is a surface area between the coolant and the steam sterilant; and
R is a heat resistance between the coolant and the steam sterilant.

With reference to FIGS. 1-5 and 12-13, in some embodiments, the processor 500 determines the temperature of the steam sterilant "$T_{steam}$" (used in Equation 2) based at least partially on the pressure of the steam sterilant. In other words, the temperature of the steam sterilant "$T_{steam}$" within the sensor tube 202 is derived from the pressure of the steam sterilant. In some cases, the temperature of the steam sterilant "$T_{steam}$" within the sensor tube 202 may be selected as about 134 degree Celsius, which is a temperature of the steam sterilant in a holdingphase of a sterilization cycle.

In some embodiments, the processor 500 further determines the average temperature of the coolant "$T_c$" (used in Equation 2) based at least partially on the signals received from the first temperature sensor 208 and the second temperature sensor 210. The heat resistance "R" between the coolant and the steam sterilant can be derived from a material of the sensor tube 202.

The surface area "A" (used in Equation 2) between the coolant and the steam sterilant is calculated by Equation 3 provided below:

$$A = 2\pi r \cdot (h_{tube} - h_{NCG}) \qquad \text{(Equation 3)}$$

where, r is a radius of sensor tube 202;

$h_{tube}$ is the height of the sensor tube 202 as shown in FIG. 13; and
$h_{NCG}$ is the height of the sensor tube 202 filled with the non-condensable gas as shown in FIG. 13.

In some embodiments, the processor 500 determines the height of the non-condensable gas bubble "$h_{NCG}$" based at least partially on the total condensation power "$P_{steam}$", the temperature of the steam sterilant "$T_{steam}$", and the average temperature of the coolant "$T_c$". Therefore, by using the Equations 1, 2 and 3, the height of the non-condensable gas bubble "$h_{NCG}$" at a given time"t" is calculated by Equation 4 provided below:

$$h_{NCG}(t) = h_{tube} - \frac{P_{steam}}{T_{steam} - T_{water}} \cdot R \cdot \frac{1}{2\pi r} \qquad \text{(Equation 4)}$$

For the sensor tube 702 (shown in FIGS. 7 and 8), a height of the non-condensable gas bubble "$h_{NCG}$" may be determined by a thermal image produced by the thermal imaging sensor 768 (shown in FIGS. 8 and 9).

By using the height of the non-condensable gas bubble "$h_{NCG}$" and a cross sectional area of the sensor tube 202, a volume of the non-condensable gas "$V_{NCG}$" at a given time "t" is calculated by Equation 5 provided below:

$$V_{NCG}(t) = h_{NCG} \cdot \pi r^2 \qquad \text{(Equation 5)}$$

In some embodiments, the processor 500 determines a molar amount of the non-condensable gas bubble "$N_{NCG}$" based at least partially on the height of the non-condensable gas bubble "$h_{NCG}$", the pressure of the steam sterilant, and a temperature of the non-condensable gas bubble "$T_{NCG}$". The temperature of the non-condensable gas bubble "$T_{NCG}$" may be determined based on the signal received from the first temperature sensor 208. In other words, the temperature of the non-condensable gas bubble "$T_{NCG}$" may be determined based on the temperature of the coolant proximal to the inlet 252 of the housing 250. By using ideal gas law, the molar amount of the non-condensable gas bubble "$N_{NCG}$" at a given time "t" can be calculated by Equation 6 provided below:

$$N_{NCG}(t) = \frac{PV_{NCG}}{RT_{NCG}} \qquad \text{(Equation 6)}$$

where, "P" is the pressure of the steam sterilant within the sensor tube 202; and
"R" is gas constant.

Further, by using the total condensation power of the steam sterilant "$P_{steam}$", a molar condensation rate of the coolant "$dN_c / dt$" at a given time "t" can be calculated by Equation 7 provided below:

$$\frac{dN_c}{dt}(t) = \frac{P_{steam}}{Q_{condensation}} \qquad \text{(Equation 7)}$$

where, $Q_{condensation}$ is condensation energy per mol of the steam sterilant.

In some embodiments, the processor 500 determines the steam quality of the steam sterilant based at least partially on the molar amount of the non-condensable gas bubble "$N_{NCG}$" and the total condensation power of the steam sterilant "$P_{steam}$". Specifically, for a given time period, the steam quality of the steam sterilant is determined by using the molar amount of the non-condensable gas bubble "$N_{NCG}$" and the molar condensation rate of the coolant "$dNc / dt$". For a specific time period from a time "t1" to a time "t2", the steam quality of the steam sterilant is calculated using Equation 6 and Equation 7. The steam quality of the steam sterilant is calculated according to Equation 8 provided below:

$$\eta(t) = \frac{N_{NCG}(t2) - N_{NCG}(t1)}{\int_{t1}^{t2} \frac{dNc}{dt} dt} \qquad \text{(Equation 8)}$$

where, $\eta$ is a steam quality of the steam sterilant.

In some embodiments, the processor 500 is further configured to determine a result of the Bowie-Dick test based on the steam quality "$\eta$" of the steam sterilant. In some cases, the sensing devices 200, 700 may use an algorithm to determine a result of the Bowie-Dick test based on information pertaining to the steam quality "$\eta$". The Bowie-Dick test result may inform an operator whether the steam quality "$\eta$" is sufficient to sterilize various medical equipment. Generally, the Bowie-Dick test is conducted at the start of each day before using a given steam sterilizer.

Referring to FIGS. 1-3 and 6-9, as the sensing devices 200, 700 are integral parts of the sterilizing systems 100, 600, respectively, a user may determine the steam quality of the steam sterilant or efficacy of the air removal stage of steam sterilization process in an easy and convenient manner. By using an appropriate algorithm, each of the sensing devices 200, 700 may be used to conduct Bowie-Dick test to monitor the steam quality of the steam sterilant received within the steam sterilizer 102. Further, the sterilizing systems 100, 600 including the respective sensing devices 200, 700 are built-in and stand-alone systems. Therefore, the sterilizing systems 100, 600 may not require insertion of any additional components, such as indicator sheets, into the steam sterilizer 102 and then removal of the additional components to determine the steam quality. This may enable rapid testing of the steam quality of the steam sterilant. Consequently, the disclosed sterilizing systems 100, 600 including the respective sensing devices 200, 700 may increase an efficiency and decrease an overall cost and a complexity of a testing process to determine the steam quality of the steam sterilant. The sterilizing systems 100, 600 may reduce or eliminate recurring costs associated with conventional testing methods, since no disposable components are required for testing. As the sensing devices 200, 700 are coupled to the steam sterilizers 102, there may be no need for separate storage of the testing unit, i.e., the sensing devices 200, 700. Further, there may be no possibility of misplacing the sensing devices 200, 700.

Moreover, the sensing devices 200, 700 may be used for a longer time period and for a greater number of test cycles as compared to conventional test units. In some cases, there may not be any limit to the number of test cycles in which the sensing devices 200, 700 can be used. Therefore, the sterilizing systems 100, 600 including the respective sensing devices 200, 700 may be sustainable setups with low operational costs. The sensing devices 200, 700 may be used for a next test cycle immediately after a current test cycle is completed. Therefore, the sterilizing systems 100, 600 including the respective sensing devices 200, 700 may require minimal or no service/recovery time between two consecutive test cycles. Further, the mounting of the sensing devices 200, 700 outside the steam sterilizer 102 may ensure that each of the sensing devices 200, 700 does not consume any space within the steam sterilizer 102. In general, a space within the steam sterilizer 102 may be optimally utilized for receiving a load or medical equipment.

Further, the sensing devices 200, 700 including the respective sensor tubes 202, 702 may be provided a greater cooling capacity by the coolant within the respective flow passages 256, 756. In some cases, the coolant flowing with the respective flow passages 256, 756 may provide an unlimited heat capacity to the respective sensor tubes 202, 702. Consequently, dimensions of the respective sensor tubes 202, 702 may be increased as per application requirements to achieve an improved testing sensitivity without any limitations being imposed by possible overheating. This may improve an overall sensitivity of the sensing devices 200, 700 to the steam quality of the steam sterilant received within the respective sensor tubes 202, 702. Therefore, the sensing devices 200, 700 may provide more accurate and precise test results while monitoring the steam quality of the steam sterilant.

Additionally, one or more output signals of the sensing devices 200, 700 may be quantitatively related to the steam quality of the steam sterilant. Therefore, the sensing devices 200, 700 may provide quantitative measurements related to the steam quality as opposed to a mere pass or fail indication. Such quantitative measurements can help in detecting possible causes of low steam quality.

Furthermore, most of the commercially available steam sterilizers already include one or two lead-ins extending from a sidewall or a top wall of the chamber. Thus, the sensing devices 200, 700 may be retrofitted to steam sterilizers that are already manufactured and are being currently used in the medical industry.

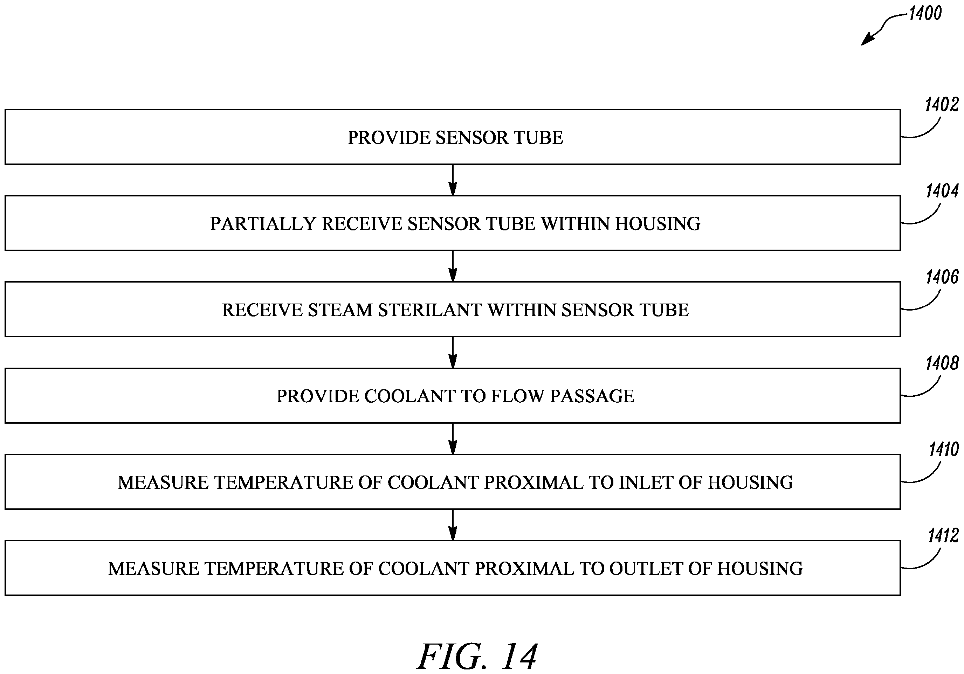

FIG. 14 illustrates a flow chart for a method 1400 for monitoring a stem quality of a steam sterilant in the chamber 104 (shown in FIGS. 1 and 6). With reference to FIGS. 4 and 7, at step 1402, the method 1400 includes providing the sensor tubes 202, 702 having respective open ends 204, 704 and respective closed ends 206, 706 opposite to the respective open ends 204, 704.

Referring to FIGS. 2, 3, 8 and 9, at step 1404, the method 1400 includes at least partially receiving the sensor tubes 202, 702 within respective housings 250, 750. The housing 250 includes the inlet 252 and the outlet 254 spaced apart from the inlet 252. The housing 750 includes the inlet 752 and the outlet 754 spaced apart from the inlet 752. At step 1406, the method 1400 includes receiving the steam sterilant within the sensor tubes 202, 702 via the respective open ends 204, 704.

Referring to FIGS. 2, 3 and 8, at step 1408, the method 1400 includes providing a coolant, via the respective inlets 252, 752, to respective flow passages 256, 756 defined by the respective housings 250, 750. The flow passage 256 (shown in FIGS. 2 and 3) extends at least partially along the length of the sensor tube 202 between the inlet 252 and the outlet 254. The flow passage 756 (shown in FIG. 8) extends at least partially along the length of the sensor tube 702 between the inlet 752 and the outlet 754.

Referring to FIGS. 1-5 and 7-9, at step 1410, the method 1400 includes measuring, by the first temperature sensor 208 disposed in fluid communication with the flow passages 256, 756, a temperature of the coolant proximal to the respective inlets 252, 752 of the respective housings 250, 750. At step 1412, the method 1400 includes measuring, by the second temperature sensor 210 disposed in fluid communication with the flow passages 256, 756, a temperature of the coolant proximal to the respective outlets 254, 754 of the respective housings 250, 750.

Referring to FIGS. 2-3 and 7-9, in some embodiments, the method 1400 further includes measuring, by the flow sensor 216 disposed in fluid communication with the flow passages 256, 756, a flow rate of the coolant flowing through the flow passages 256, 756. In some embodiments, the method 1400 further includes measuring, by the pressure sensor 214 disposed in fluid communication with the sensor tubes 202, 702, the pressure of the steam sterilant within the sensor tubes 202, 702.

Referring to FIGS. 2, 3, 7, 9, and 12, in some embodiments, the method 1400 further includes determining, by the processor 500, the steam quality (using Equation 8) of the steam sterilant based at least partially on the pressure of the steam sterilant and the signals received from the first temperature sensor 208, the second temperature sensor 210, and the flow sensor 216. The processor 500 is communicably coupled to the first temperature sensor 208, the second temperature sensor 210, and the flow sensor 216.

In some embodiments, determining the steam quality of the steam sterilant further includes determining, by the processor 500, the total condensation power of the steam sterilant "$P_{steam}$" based at least partially on the signals received from the first temperature sensor 208, the second temperature sensor 210, and the flow sensor 216.

In some embodiments, determining the steam quality of the steam sterilant further includes determining, by the processor 500, the temperature of the steam sterilant "$T_{steam}$" based at least partially on the pressure of the steam sterilant. In some embodiments, determining the steam quality of the steam sterilant further includes determining the average temperature of the coolant "$T_c$" based at least partially on the signals received from the first temperature sensor 208 and the second temperature sensor 210.

Referring to FIGS. 12 and 13, in some embodiments, determining the steam quality of the steam sterilant further includes determining, by the processor 500, the height of a non-condensable gas bubble "$h_{NCG}$" (using Equation 4) based at least partially on the total condensation power "$P_{steam}$", the temperature of the steam sterilant "$T_{steam}$", and the average temperature of the coolant "$T_c$".

In some embodiments, determining the steam quality of the steam sterilant further includes determining the molar amount of the non-condensable gas bubble "$N_{NCG}$" (using Equation 6) based at least partially on the height of the non-condensable gas bubble "$h_{NCG}$", the pressure of the steam sterilant, and the temperature of the non-condensable gas bubble "$T_{NCG}$".

In some embodiments, the method 1400 further includes determining the steam quality (using Equations 7 and 8) of the steam sterilant based at least partially on the molar amount of the non-condensable gas bubble "$N_{NCG}$" and the total condensation power of the steam sterilant "$P_{steam}$".

Referring to FIGS. 1-5, in some embodiments, the method 1400 further includes determining, by the at least one intermediate temperature sensor 212 disposed in fluid communication with the flow passage 256 between the first temperature sensor 208 and the second temperature sensor 210, a temperature of the coolant between the inlet 252 and the outlet 254.

Referring to FIGS. 6-9, in some embodiments, the method 1400 further includes detecting, by the thermal imaging sensor 768 coupled to the housing 750, a thermal profile of the sensor tube 702. In some embodiments, the method 1400 further includes generating, by the fan 760 disposed within the housing 750 proximal to the outlet 754, a flow of the coolant through the housing 750.

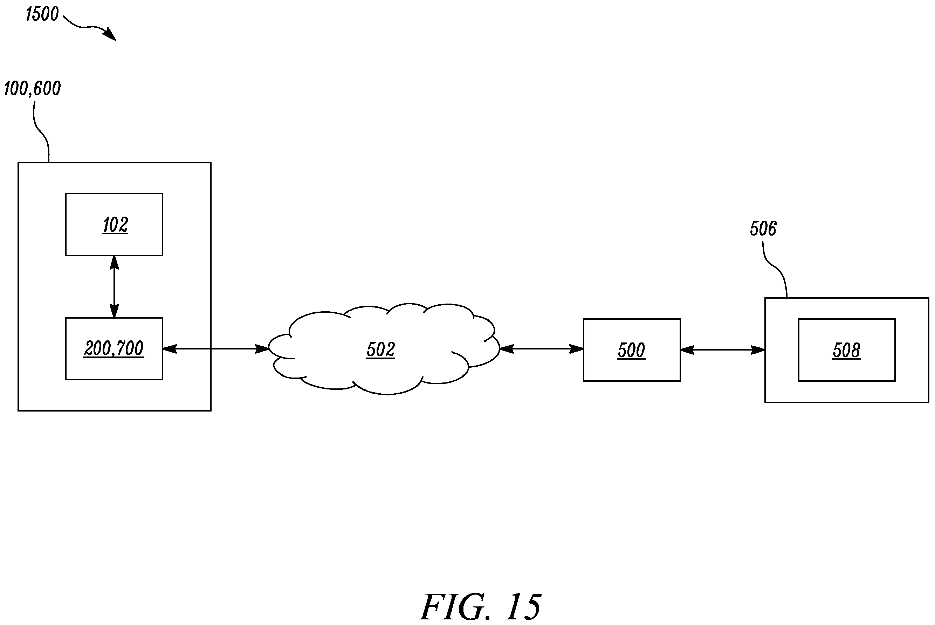

FIG. 15 illustrates a cloud system 1500 according to an embodiment of the present disclosure. The cloud system includes the sterilizing systems 100, 600, the processor 500, and a memory 506. The processor 500 is communicably coupled to the sensing devices 200, 700 of the respective sterilizing systems 100, 600. Specifically, with reference to FIGS. 1-5 and 7-9, the processor is communicably coupled to the first temperature sensor 208, the second temperature sensor 210, and the flow sensor 216. The processor 500 is further communicably coupled to the memory 506.

The memory 506 includes a database 508. The database 508 may have a data structure to store organized information and data. The memory 506 may also include a suitable combination of computing devices and software modules for storing, manipulating, and retrieving data records related to the sensing devices 200, 700, and the sterilizing systems 100, 600.

The processor 500 may store data received from the first temperature sensor 208, the second temperature sensor 210, and the flow sensor 216 in the database 508. The processor 500 may further retrieve the same data which was stored in the database 508 of the memory 506.

Further, once the processor 500 determines a steam quality (using Equation 8) of a steam sterilant, a data or an information pertaining to the steam quality is analyzed by the processor 500 and then stored in the database 508. A test result indicating the steam quality of the steam sterilant may be transmitted or passed to a concerned department, e.g., a hospital, a medical lab, etc. The department can check the test result on one or more devices with required login credentials. Further, an algorithm in the processor 500 may also be updated with time based on the test results over a period of time. The processor 500 may also generate a trend analysis report that can be shared with a concerned department, e.g., a hospital, a medical lab, etc. In an example, the trend analysis report may show a graph depicting a trend of results in which the steam quality was satisfactory.

In some cases, the cloud system 1500 may be able to determine a number of test cycles run in a given time period. This may help to manufacture the sensing devices 200, 700 in such a way that the sensing devices 200, 700 may be able to run for a minimum number of test cycles in a given time period.

Unless otherwise indicated, all numbers expressing feature sizes, amounts, and physical properties used in the specification and aspects are to be understood as being modified by the term "about". Accordingly, unless indicated to the contrary, the numerical parameters set forth in the foregoing specification and attached aspects are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. This application is intended to cover any adaptations or variations of the specific embodiments discussed herein. Therefore, it is intended that this disclosure be limited only by the aspects and the equivalents thereof.

The following aspects are preferred embodiments of the invention:

1. A sensing device for monitoring a steam quality of a steam sterilant in a chamber, the sensing device comprising:

a sensor tube having an open end and a closed end opposite to the open end, wherein the open end is disposed in fluid communication with the chamber and configured to receive the steam sterilant from the chamber;
a housing at least partially receiving the sensor tube therein and surrounding at least a portion of the sensor tube between the open end and the closed end, the housing comprising an inlet and an outlet spaced apart from the inlet, wherein the housing defines a flow passage at least partially along a length of the sensor tube between the inlet and the outlet, and wherein the inlet of the housing is configured to receive a coolant within the flow passage and the outlet of the housing is configured to discharge the coolant from the flow passage;
a first temperature sensor disposed in fluid communication with the flow passage and configured to measure a temperature of the coolant proximal to the inlet of the housing; and
a second temperature sensor disposed in fluid communication with the flow passage and configured to measure a temperature of the coolant proximal to the outlet of the housing.

2. The sensing device of aspect 1, further comprising a flow sensor disposed in fluid communication with the flow passage and configured to measure a flow rate of the coolant flowing through the flow passage.

3. The sensing device of aspect 2, further comprising a processor communicably coupled to the first temperature sensor, the second temperature sensor, and the flow sensor, wherein the processor is configured to determine the steam quality of the steam sterilant based at least partially on a pressure of the steam sterilant and signals received from the first temperature sensor, the second temperature sensor, and the flow sensor.

4. The sensing device of aspect 3, further comprising a pressure sensor disposed in fluid communication with the sensor tube and configured to measure a pressure of the steam sterilant within the sensor tube, and wherein the processor is configured to determine the pressure of the steam sterilant based on signals received from the pressure sensor.

5. The sensing device of aspect 3, wherein the processor is further configured to determine a result of a Bowie-Dick test based on the steam quality of the steam sterilant.

6. The sensing device of any one of the preceding aspects, wherein the first temperature sensor and the second temperature sensor are spaced apart from each other along the length of the sensor tube.

7. The sensing device of any one of the preceding aspects, further comprising an insulation layer disposed at least partially around the sensor tube between the open end and the closed end, wherein the insulation layer comprises a thermally insulating material.

8. The sensing device of any one of the preceding aspects, wherein housing comprises a coolant jacket.

9. The sensing device of any one of the preceding aspects, wherein the coolant comprises a liquid or a gas.

10. The sensing device of any one of the preceding aspects, wherein the inlet of the housing is disposed proximal to the closed end of the sensor tube and the outlet of the housing is disposed proximal to the open end of the sensor tube.

11. The sensing device of any one of the preceding aspects, further comprising at least one intermediate temperature sensor disposed in fluid communication with the flow passage between the first temperature sensor and the second temperature sensor, wherein the at least intermediate temperature sensor is configured to measure the temperature of the coolant.

12. The sensing device of any one of the preceding aspects, further comprising a plurality of laminar plates spaced apart from each other along a length of the sensor tube, wherein each laminar plate is engaged with and disposed around the sensor tube, and wherein each laminar plate defines a plurality of apertures therethrough.

13. The sensing device of aspect 11, wherein the at least one intermediate temperature sensor comprises a plurality of intermediate temperature sensors, and wherein each intermediate temperature sensor is fluidly disposed between two corresponding adjacent laminar plates.

14. The sensing device of any one of the preceding aspects, further comprising a thermal imaging sensor coupled to the housing and configured to detect a thermal profile of the sensor tube.

15. The sensing device of aspect 14, wherein the thermal imaging sensor is an infrared camera.

16. The sensing device of aspect 14 or 15, wherein the housing further comprises:

a first portion comprising the inlet and the outlet, the first portion at least partially receiving the sensor tube therein; and
a second portion extending from the first portion, the second portion comprising a sensor end distal to the first portion;
wherein the thermal imaging sensor is disposed at the sensor end.

17. The sensing device of aspect 16, wherein the second portion of the housing tapers from the first portion to the sensor end.

18. The sensing device of any one of the preceding aspects, further comprising an insulating case at least partially receiving the housing therein, wherein the insulating case comprises a thermally insulating material.

19. The sensing device of any one of the preceding aspects, wherein the sensor tube comprises a main portion comprising the closed end and an inlet portion extending from the main portion and comprising the open end, and wherein the inlet portion is inclined relative to the main portion.

20. The sensing device of aspect 19, wherein the main portion is at least partially surrounded by the housing and the inlet portion extends at least partially through the housing.

21. The sensing device of any one of the preceding aspects, further comprising a fan disposed within the housing proximal to the outlet, wherein the fan is configured to generate a flow of the coolant through the housing.

22. The sensing device of any one of the preceding aspects, further comprising a plurality of cooling fins disposed on the sensor tube at least partly along the length of the sensor tube.

23. A sterilizing system comprising:

a steam sterilizer defining a chamber therein, wherein the chamber is configured to receive a steam sterilant therein; and
the sensing device of aspect 1, wherein the sensor tube of the sensing device is coupled to the steam sterilizer, such that the open end of the sensor tube is disposed in fluid communication with the chamber of the steam sterilizer.

24. The sterilizing system of aspect 23, wherein the steam sterilizer comprises a top wall, a bottom wall opposite to the top wall, and a pair of side walls extending between the top wall and the bottom wall, and wherein the top wall, the bottom wall and the side walls define the chamber therebetween, and wherein the sensor tube is coupled to the top wall or one of the sidewalls.

25. A method for monitoring a steam quality of a steam sterilant in a chamber, the method comprising:

providing a sensor tube having an open end and a closed end opposite to the open end;
at least partially receiving the sensor tube within a housing, wherein the housing comprises an inlet and an outlet spaced apart from the inlet;
receiving the steam sterilant within the sensor tube via the open end;
providing a coolant, via the inlet, to a flow passage defined by the housing, wherein the flow passage extends at least partially along a length of the sensor tube between the inlet and the outlet;
measuring, by a first temperature sensor disposed in fluid communication with the flow passage, a temperature of the coolant proximal to the inlet of the housing; and
measuring, by a second temperature sensor disposed in fluid communication with the flow passage, a temperature of the coolant proximal to the outlet of the housing.

26. The method of aspect 25, further comprising measuring, by a flow sensor disposed in fluid communication with the flow passage, a flow rate of the coolant flowing through the flow passage.

27. The method of aspect 26, further comprising determining, by a processor, the steam quality of the steam sterilant based at least partially on a pressure of the steam sterilant and signals received from the first temperature sensor, the second temperature sensor, and the flow sensor, wherein the processor is communicably coupled to the first temperature sensor, the second temperature sensor, and the flow sensor.

28. The method of aspect 27, further comprising measuring, by a pressure sensor disposed in fluid communication with the sensor tube, a pressure of the steam sterilant within the sensor tube.

29. The method of aspect 27 or 28, further comprising determining, by the processor, a result of a Bowie-Dick test based on the steam quality of the steam sterilant.

30. The method of aspect 27, 28, or 29, wherein determining the steam quality of the steam sterilant further comprises determining, by the processor, a total condensation power of the steam sterilant based at least partially on the signals received from the first temperature sensor, the second temperature sensor, and the flow sensor.

31. The method of aspect 30, wherein determining the steam quality of the steam sterilant further comprises:

determining, by the processor, a temperature of the steam sterilant based at least partially on the pressure of the steam sterilant;
determining an average temperature of the coolant based at least partially on the signals received from the first temperature sensor and the second temperature sensor;
determining, by the processor, a height of a non-condensable gas bubble based at least partially on the total condensation power, the temperature of the steam sterilant, and the average temperature of the coolant; and
determining a molar amount of the non-condensable gas bubble based at least partially on the height of the non-condensable gas bubble, the pressure of the steam sterilant, and a temperature of the non-condensable gas bubble.

32. The method of aspect 31, further comprising determining the steam quality of the steam sterilant based at least partially on the molar amount of the non-condensable gas bubble and the total condensation power of the steam sterilant.

33. The method of any one of aspects 25-32, further comprising determining, by at least one intermediate temperature sensor disposed in fluid communication with the flow passage between the first temperature sensor and the second temperature sensor, a temperature of the coolant between the inlet and the outlet.

34. The method of any one of aspects 25-33, further comprising detecting, by a thermal imaging sensor coupled to the housing, a thermal profile of the sensor tube.

35. The method of any one of aspects 25-34, further comprising generating, by a fan disposed within the housing proximal to the outlet, a flow of the coolant through the housing.

**Claims**

1. A sensing device for monitoring a steam quality of a steam sterilant in a chamber, the sensing device comprising:

a sensor tube having an open end and a closed end opposite to the open end, wherein the open end is disposed in fluid communication with the chamber and configured to receive the steam sterilant from the chamber;
a housing at least partially receiving the sensor tube therein and surrounding at least a portion of the sensor tube between the open end and the closed end, the housing comprising an inlet and an outlet spaced apart from the inlet, wherein the housing defines a flow passage at least partially along a length of the sensor tube between the inlet and the outlet, and wherein the inlet of the housing is configured to receive a coolant within the flow passage and the outlet of the housing is configured to discharge the coolant from the flow passage;

a first temperature sensor disposed in fluid communication with the flow passage and configured to measure a temperature of the coolant proximal to the inlet of the housing; and

a second temperature sensor disposed in fluid communication with the flow passage and configured to measure a temperature of the coolant proximal to the outlet of the housing.

2. The sensing device of claim 1, further comprising a flow sensor disposed in fluid communication with the flow passage and configured to measure a flow rate of the coolant flowing through the flow passage.

3. The sensing device of claim 2, further comprising a processor communicably coupled to the first temperature sensor, the second temperature sensor, and the flow sensor, wherein the processor is configured to determine the steam quality of the steam sterilant based at least partially on a pressure of the steam sterilant and signals received from the first temperature sensor, the second temperature sensor, and the flow sensor.

4. The sensing device of claim 3, further comprising a pressure sensor disposed in fluid communication with the sensor tube and configured to measure a pressure of the steam sterilant within the sensor tube, and wherein the processor is configured to determine the pressure of the steam sterilant based on signals received from the pressure sensor.

5. The sensing device of claim 3, wherein the processor is further configured to determine a result of a Bowie-Dick test based on the steam quality of the steam sterilant.

6. The sensing device of any one of the preceding claims, wherein the first temperature sensor and the second temperature sensor are spaced apart from each other along the length of the sensor tube.

7. The sensing device of any one of the preceding claims, further comprising an insulation layer disposed at least partially around the sensor tube between the open end and the closed end, wherein the insulation layer comprises a thermally insulating material.

8. The sensing device of any one of the preceding claims, wherein housing comprises a coolant jacket.

9. The sensing device of any one of the preceding claims, wherein the coolant comprises a liquid or a gas.

10. The sensing device of any one of the preceding claims, wherein the inlet of the housing is disposed proximal to the closed end of the sensor tube and the outlet of the housing is disposed proximal to the open end of the sensor tube.

11. A sterilizing system comprising:

a steam sterilizer defining a chamber therein, wherein the chamber is configured to receive a steam sterilant therein; and

the sensing device of claim 1, wherein the sensor tube of the sensing device is coupled to the steam sterilizer, such that the open end of the sensor tube is disposed in fluid communication with the chamber of the steam sterilizer.

12. A method for monitoring a steam quality of a steam sterilant in a chamber, the method comprising:

providing a sensor tube having an open end and a closed end opposite to the open end;
at least partially receiving the sensor tube within a housing, wherein the housing comprises an inlet and an outlet spaced apart from the inlet;
receiving the steam sterilant within the sensor tube via the open end;
providing a coolant, via the inlet, to a flow passage defined by the housing, wherein the flow passage extends at least partially along a length of the sensor tube between the inlet and the outlet;
measuring, by a first temperature sensor disposed in fluid communication with the flow passage, a temperature of the coolant proximal to the inlet of the housing; and
measuring, by a second temperature sensor disposed in fluid communication with the flow passage, a temperature of the coolant proximal to the outlet of the housing.

13. The method of claim 12, further comprising measuring, by a flow sensor disposed in fluid communication with the flow passage, a flow rate of the coolant flowing through the flow passage.

**14.** The method of claim 13, further comprising determining, by a processor, the steam quality of the steam sterilant based at least partially on a pressure of the steam sterilant and signals received from the first temperature sensor, the second temperature sensor, and the flow sensor, wherein the processor is communicably coupled to the first temperature sensor, the second temperature sensor, and the flow sensor.

**15.** The method of claim 14, further comprising measuring, by a pressure sensor disposed in fluid communication with the sensor tube, a pressure of the steam sterilant within the sensor tube.

*FIG. 1*

FIG. 2

FIG. 3

**FIG. 4**

EP 4 083 587 A1

**FIG. 5**

*FIG. 6*

23

*FIG. 7*

*FIG. 8*

EP 4 083 587 A1

FIG. 9

EP 4 083 587 A1

*FIG. 10*

*FIG. 11*

*FIG. 12*

EP 4 083 587 A1

**FIG. 13**

1400

```
┌─────────────────────────────────────────────────────────┐  1402
│                  PROVIDE SENSOR TUBE                      │
└─────────────────────────────────────────────────────────┘
                            │
                            ▼
┌─────────────────────────────────────────────────────────┐  1404
│         PARTIALLY RECEIVE SENSOR TUBE WITHIN HOUSING       │
└─────────────────────────────────────────────────────────┘
                            │
                            ▼
┌─────────────────────────────────────────────────────────┐  1406
│          RECEIVE STEAM STERILANT WITHIN SENSOR TUBE        │
└─────────────────────────────────────────────────────────┘
                            │
                            ▼
┌─────────────────────────────────────────────────────────┐  1408
│              PROVIDE COOLANT TO FLOW PASSAGE               │
└─────────────────────────────────────────────────────────┘
                            │
                            ▼
┌─────────────────────────────────────────────────────────┐  1410
│   MEASURE TEMPERATURE OF COOLANT PROXIMAL TO INLET OF HOUSING │
└─────────────────────────────────────────────────────────┘
                            │
                            ▼
┌─────────────────────────────────────────────────────────┐  1412
│  MEASURE TEMPERATURE OF COOLANT PROXIMAL TO OUTLET OF HOUSING │
└─────────────────────────────────────────────────────────┘
```

*FIG. 14*

*FIG. 15*

Europäisches
Patentamt

European
Patent Office

Office européen
des brevets

# EUROPEAN SEARCH REPORT

Application Number

EP 22 17 0849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 625 553 A1 (SOLIDTOO B V [NL]) 25 March 2020 (2020-03-25) * paragraphs [0001], [0017] – [0019], [0021], [0024], [0026], [0035], [0037]; figures 1-4 * | 1-15 | INV. G01K13/02 G01J5/00 G01K1/02 G01N7/00 G01N33/00 G01F23/00 G01K17/10 A61L2/07 A61L2/28 |
| A | US 2015/224216 A1 (SCHUMACHER KNUT [DE] ET AL) 13 August 2015 (2015-08-13) * paragraphs [0001], [0002], [0012], [0015], [0032], [0034], [0048], [0188]; figures 4,5 * | 1-15 | |

TECHNICAL FIELDS
SEARCHED       (IPC)

G01K
G01J
G01N
G01F

The present search report has been drawn up for all claims

| Place of search | Date of completion of the search | Examiner |
|---|---|---|
| The Hague | 6 September 2022 | Phleps, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 0849

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2022

| Patent document cited in search report | | Publication date | Patent family member(s) | | Publication date |
|---|---|---|---|---|---|
| EP 3625553 | A1 | 25-03-2020 | EP | 3625553 A1 | 25-03-2020 |
| | | | ES | 2884774 T3 | 13-12-2021 |
| | | | US | 2021154346 A1 | 27-05-2021 |
| | | | WO | 2018210850 A1 | 22-11-2018 |
| US 2015224216 | A1 | 13-08-2015 | BR | 112015006044 A2 | 22-05-2018 |
| | | | CA | 2885258 A1 | 27-03-2014 |
| | | | CN | 104812417 A | 29-07-2015 |
| | | | EP | 2897652 A1 | 29-07-2015 |
| | | | JP | 6199976 B2 | 20-09-2017 |
| | | | JP | 2015534478 A | 03-12-2015 |
| | | | KR | 20150058338 A | 28-05-2015 |
| | | | US | 2015224216 A1 | 13-08-2015 |
| | | | WO | 2014046998 A1 | 27-03-2014 |

EPO FORM P0459

For more details about this annex : see Official Journal of the European Patent Office, No. 12/82